# EUROPEAN PATENT APPLICATION

(11) **EP 4 528 651 A1**
(43) Date of publication of application: **26.03.2025**
(21) Application number: 24201883.6
(22) Date of filing: 23.09.2024
(51) Int. Cl.: G06T 11/00

(54) **IMAGE PROCESSING APPARATUS, RADIOGRAPHY SYSTEM, AND PROGRAM**

(30) Priority: 25.09.2023 JP 2023160710
(71) Applicant: FUJIFILM Corporation, Tokyo 106-8620 (JP)
(72) Inventor: FUKUDA, Wataru, Kanagawa 258-8538 (JP)
(74) Representative: Dehns Germany Partnerschaft mbB

(57) **Abstract**

An image processing apparatus includes at least one processor that is configured to: acquire projection images obtained by imaging a subject with radiation at a plurality of angles; reconstruct a plurality of tomographic images from the acquired projection images; detect a structure of interest from the plurality of reconstructed tomographic images; derive a region of the projection image corresponding to the detected structure of interest; and determine, by using the derived region of the projection image, whether or not the tomographic image from which the structure of interest is detected is a tomographic image corresponding to a focal plane of the structure of interest.

## Description

### BACKGROUND

### Technical Field

The present disclosure relates to an image processing apparatus, a radiography system, an image processing method and a non-transitory storage medium storing a program.

### Related Art

In recent years, image diagnosis using a radiography apparatus (called mammography) for capturing an image of a breast has attracted attention in order to promote early detection of breast cancer. Further, in the mammography, tomosynthesis imaging has been proposed which moves a radiation source, irradiates the breast with radiation at a plurality of radiation source positions to acquire a plurality of projection images, and reconstructs the plurality of acquired projection images to generate tomographic images in which desired tomographic planes have been emphasized. In the tomosynthesis imaging, the plurality of projection images are acquired by imaging the breast at the plurality of radiation source positions by moving the radiation source in parallel with a radiation detector or moving the radiation source to draw a circle or an elliptical arc in accordance with characteristics of an imaging apparatus and a required tomographic image, and the projection images are reconstructed using a back projection method such as a simple back projection method or a filtered back projection method or a sequential reconstruction method. Thereby, a tomographic image is generated.

The tomographic images are generated in a plurality of tomographic planes of the breast, which makes it possible to separate structures that overlap each other in the depth direction in which the tomographic planes are arranged in the breast. Therefore, it is possible to find an abnormal part such as a lesion that has been difficult to detect in a two-dimensional image (hereinafter, referred to as a simple two-dimensional image) acquired by simple imaging according to the related art which irradiates a subject with radiation in a predetermined direction.

As a technology using the tomographic images in this way, JP 2014-128716 A discloses a technology of generating a pseudo two-dimensional image (hereinafter, referred to as a synthesized two-dimensional image) corresponding to a simple two-dimensional image by synthesizing a plurality of tomographic images having different distances (positions in a height direction) from a detection surface of a radiation detector to a radiation source, using an addition method, an averaging method, a maximum intensity projection method, a minimum intensity projection method, or the like, the tomographic images being acquired by tomosynthesis imaging.

Further, JP 6185023 B discloses a tomographic image generation apparatus that is intended to improve a quality of a tomographic image while shortening an operation time in a case of generating tomographic images from a plurality of projection images acquired by performing imaging at a plurality of radiation source positions, such as tomosynthesis imaging.

The tomographic image generation apparatus comprises an image acquisition unit that acquires a plurality of projection images corresponding to a plurality of radiation source positions, the projection images being obtained by relatively moving a radiation source with respect to a detection unit and irradiating a subject with radiation at the plurality of radiation source positions obtained by the movement of the radiation source. In addition, the tomographic image generation apparatus comprises a pixel value projection unit that projects pixel values of the plurality of projection images to coordinate positions on a desired tomographic plane of the subject based on a positional relationship between the radiation source position when imaging each of the plurality of projection images and the detection unit while holding the pixel values of the plurality of projection images. Further, the tomographic image generation apparatus comprises a pixel value calculation unit that generates a tomographic image of the tomographic plane by calculating a pixel value at a coordinate position of interest based on the plurality of pixel values of the projection images projected in a predetermined range which is determined in advance with the coordinate position of interest on the tomographic plane as a reference.

Meanwhile, in a case of creating, from the tomographic images, a synthesized image such as a synthesized two-dimensional image or a slab image which is an image including information of a certain range in a height direction, that is, an image including thickness information, it is required to create a synthesized image that provides high visibility for a structure of interest, such as a tumor, a spicula, or a calcification.

For this reason, it is desired to specify a tomographic image in which the structure of interest is actually located (hereinafter, referred to as a "tomographic image corresponding to a focal plane") and to create a synthesized image in which the specified tomographic image is emphasized. However, in the technologies disclosed in JP 2014-128716 A and JP 6185023 B, this point is not considered, and it is not possible to determine the tomographic image. It is ideal that the "focal plane" is a tomographic plane corresponding to a central height at which the structure of interest is actually located or a tomographic plane in which the structure of interest is most easily determined as compared with other tomographic planes, among the tomographic planes in which the structure of interest is actually located. However, the focal plane is not limited thereto.

In addition, a method of determining a tomographic image corresponding to the focal plane from the tomographic image itself can also be considered. However, in this method, there is a problem in that it is difficult to distinguish a structure on a non-focal plane and an actual structure of interest and it is not possible to accurately determine a tomographic image corresponding to the focal plane.

### SUMMARY

The present disclosure has been made in view of the above circumstances, and an object of the present disclosure is to provide an image processing apparatus, a radiography system, an image processing method and a non-transitory storage medium storing a program capable of performing determination with higher accuracy as compared with a case of determining a tomographic image including the structure of interest and corresponding to the focal plane from the tomographic images.

According to a first aspect of the present disclosure, there is provided an image processing apparatus comprising: at least one processor, in which the processor is configured to: acquire projection images obtained by imaging a subject with radiation at a plurality of angles; reconstruct a plurality of tomographic images from the acquired projection images; detect a structure of interest from the plurality of reconstructed tomographic images; derive a region of the projection image corresponding to the detected structure of interest; and determine, by using the derived region of the projection image, whether or not the tomographic image from which the structure of interest is detected is a tomographic image corresponding to a focal plane of the structure of interest.

According to a second aspect of the present disclosure, in the image processing apparatus according to the first aspect, the processor is configured to: create a synthesized two-dimensional image from the plurality of tomographic images by using a result of the determination.

According to a third aspect of the present disclosure, in the image processing apparatus according to the first aspect, the processor is configured to: create a slab image from the plurality of tomographic images by using a result of the determination.

According to a fourth aspect of the present disclosure, in the image processing apparatus according to the first aspect, the processor is configured to: perform display of linking a synthesized two-dimensional image created from the plurality of tomographic images and the corresponding tomographic image by using a result of the determination.

According to a fifth aspect of the present disclosure, in the image processing apparatus according to the first aspect, the processor is configured to: detect the structure of interest by using at least one of a machine learning model or pattern matching using a template image of the target structure of interest.

According to a sixth aspect of the present disclosure, in the image processing apparatus according to the first aspect, the processor is configured to: perform the determination by using a similarity between the regions of the corresponding projection images.

According to a seventh aspect of the present disclosure, in the image processing apparatus according to the sixth aspect, the similarity is a correlation value between the regions of the corresponding projection images.

According to an eighth aspect of the present disclosure, in the image processing apparatus according to the sixth aspect or the seventh aspect, the processor is configured to: determine, for each type of the structure of interest, that the tomographic image at a height at which the corresponding similarity has a maximum value, among the tomographic images from which the structure of interest is detected and which are at heights, is the tomographic image corresponding to the focal plane.

According to a ninth aspect of the present disclosure, in the image processing apparatus according to the first aspect, the structure of interest is at least one of a tumor, a spicula, or a calcification.

In addition, in order to achieve the above obj ect, according to a tenth aspect of the present disclosure, there is provided a radiography system comprising: the image processing apparatus according to the first aspect; and a mammography apparatus that acquires projection images to be used by the image processing apparatus.

Further, according to an eleventh aspect of the present disclosure, there is provided a non-transitory storage medium storing a program causing a computer to execute an image processing, the image processing comprising: acquiring projection images obtained by imaging a subject with radiation at a plurality of angles; reconstructing a plurality of tomographic images from the acquired projection images; detecting a structure of interest from the plurality of reconstructed tomographic images; deriving a region of the projection image corresponding to the detected structure of interest; and determining, by using the derived region of the projection image, whether or not the tomographic image from which the structure of interest is detected is a tomographic image corresponding to a focal plane of the structure of interest.

Another aspect of the present disclosure is an image processing method executed by a computer, the method comprising: acquiring projection images obtained by imaging a subject with radiation at a plurality of angles; reconstructing a plurality of tomographic images from the acquired projection images; detecting a structure of interest from the plurality of reconstructed tomographic images; deriving a region of the projection image corresponding to the detected structure of interest; and determining, by using the derived region of the projection image, whether or not the tomographic image from which the structure of interest is detected is a tomographic image corresponding to a focal plane of the structure of interest.

According to the present disclosure, it is possible to perform determination with higher accuracy, as compared with a case of determining a tomographic image including the structure of interest and corresponding to the focal plane from the tomographic images.

### BRIEF DESCRIPTION OF THE DRAWINGS

Fig. 1 is a schematic configuration diagram of a radiography system to which an image processing apparatus according to an embodiment is applied.
Fig. 2 is a diagram illustrating a mammography apparatus according to the embodiment as viewed from a direction of an arrow A in Fig. 1.
Fig. 3 is a block diagram illustrating an example of an electrical configuration of the image processing apparatus according to the embodiment.
Fig. 4 is a functional block diagram illustrating a functional configuration of the image processing apparatus according to the embodiment.
Fig. 5 is a diagram for describing acquisition of projection images according to the embodiment.
Fig. 6 is a diagram for describing generation of tomographic images according to the embodiment.
Fig. 7 is a diagram for describing a method of determining a tomographic image corresponding to a focal plane according to the embodiment.
Fig. 8 is a diagram for describing a method of determining a tomographic image corresponding to the focal plane according to the embodiment.
Fig. 9 is a flowchart illustrating an example of image processing according to the embodiment.

### DESCRIPTION OF THE PREFERRED EMBODIMENTS

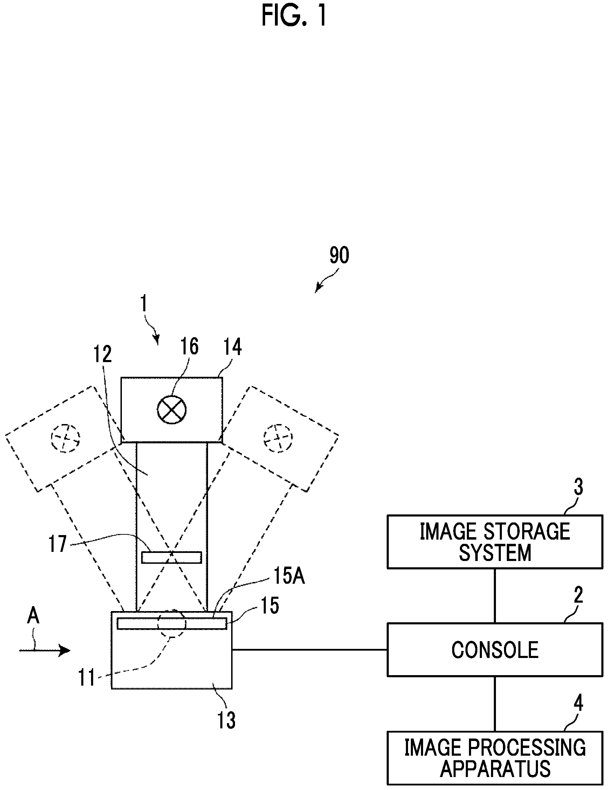

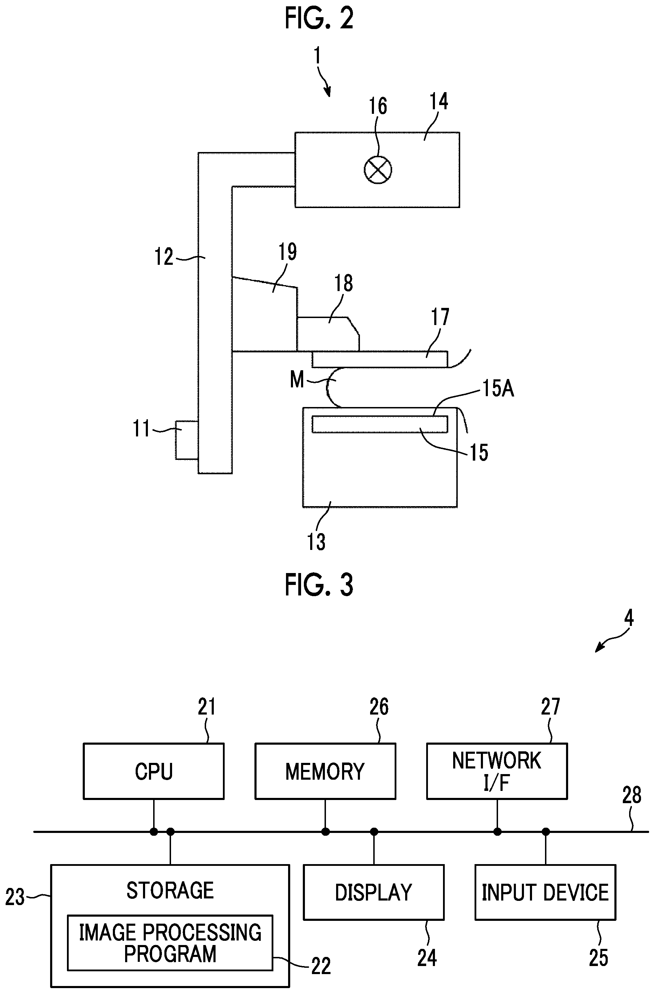

Hereinafter, an embodiment of the present disclosure will be described with reference to the drawings. Fig. 1 is a schematic configuration diagram of a radiography system 90 to which an image processing apparatus 4 according to an embodiment of the present disclosure is applied, and Fig. 2 is a diagram illustrating a mammography apparatus 1 in the radiography system 90 as viewed from a direction of an arrow A in Fig. 1.

As illustrated in Fig. 1, a radiography system 90 according to the present embodiment performs imaging of a breast M, which is a subject, at a plurality of radiation source positions and acquires a plurality of radiation images, that is, a plurality of projection images, in order to perform tomosynthesis imaging on the breast to generate tomographic images. The radiography system 90 according to the present embodiment comprises a mammography apparatus 1, a console 2, an image storage system 3, and an image processing apparatus 4.

The mammography apparatus 1 comprises an arm part 12 that is connected to a base (not illustrated) by a rotation shaft 11. An imaging table 13 is attached to one end of the arm part 12, and a radiation emitting unit 14 is attached to the other end of the arm part 12 to face the imaging table 13. The arm part 12 is configured such that only the end to which the radiation emitting unit 14 is attached can be rotated. Therefore, the imaging table 13 is fixed and only the radiation emitting unit 14 can be rotated.

A radiation detector 15, such as a flat panel detector, is provided in the imaging table 13. The radiation detector 15 has a radiation detection surface 15A. In addition, a circuit board including a charge amplifier that converts a charge signal read from the radiation detector 15 into a voltage signal, a sampling two correlation pile circuit that samples the voltage signal output from the charge amplifier, and an analog-to-digital (AD) conversion unit that converts the voltage signal into a digital signal is provided in the imaging table 13.

A radiation source 16 is accommodated in the radiation emitting unit 14. The radiation source 16 emits X-rays as radiation. The console 2 controls a timing when the radiation source 16 emits the radiation and radiation generation conditions of the radiation source 16, that is, selection of a target and filter materials, a tube voltage, an irradiation time, and the like.

Further, the arm part 12 is provided with a compression plate 17 that is disposed above the imaging table 13 and presses and compresses the breast M, a support part 18 that supports the compression plate 17, and a moving mechanism 19 that moves the support part 18 in a vertical direction in Fig. 1 and Fig. 2. A distance between the compression plate 17 and the imaging table 13, that is, a compression thickness is input to the console 2.

The console 2 has a function of controlling the mammography apparatus 1 using, for example, an imaging order and various kinds of information acquired from a radiology information system (RIS) (not illustrated) or the like through a network, such as a wireless communication local area network (LAN), and instructions or the like directly issued by an engineer or the like. Specifically, the console 2 acquires a plurality of projection images as described below by causing the mammography apparatus 1 to perform tomosynthesis imaging of the breast M. As an example, in the present embodiment, a server computer is used as the console 2.

The image storage system 3 is a system that stores image data such as the projection images which are obtained by imaging of the mammography apparatus 1. The image storage system 3 extracts an image corresponding to a request from, for example, the console 2 and the image processing apparatus 4 from the stored images, and transmits the image to an apparatus that is the source of the request. A specific example of the image storage system 3 is a picture archiving and communication system (PACS).

Next, the image processing apparatus 4 according to the present embodiment will be described. Next, a hardware configuration of the image processing apparatus 4 according to the present embodiment will be described with reference to Fig. 3. As illustrated in Fig. 3, the image processing apparatus 4 is a computer, such as a workstation, a server computer, and a personal computer, and comprises a central processing unit (CPU) 21, a non-volatile storage 23, and a memory 26 as a transitory storage region. In addition, the image processing apparatus 4 comprises a display 24 such as a liquid crystal display, an input device 25 such as a keyboard and a mouse, and a network interface (I/F) 27 connected to a network (not illustrated). The CPU 21, the storage 23, the display 24, the input device 25, the memory 26, and the network I/F 27 are connected to a bus 28. In addition, the CPU 21 is an example of a processor according to the present disclosure.

The storage 23 is implemented by a hard disk drive (HDD), a solid state drive (SSD), a flash memory, and the like. An image processing program 22 to be installed in the image processing apparatus 4 is stored in the storage 23 as a storage medium. The CPU 21 reads out the image processing program 22 from the storage 23, expands the image processing program 22 in the memory 26, and executes the expanded image processing program 22.

The image processing program 22 is stored in a storage device of a server computer connected to the network or in a network storage in a state of being accessible from the outside, and is downloaded and installed in the computer that configures the image processing apparatus 4 in response to a request. Alternatively, the image processing program 22 is distributed in a state of being recorded on a recording medium, such as a digital versatile disc (DVD) or a compact disc read only memory (CD-ROM), and is installed in the computer that configures the image processing apparatus 4 from the recording medium.

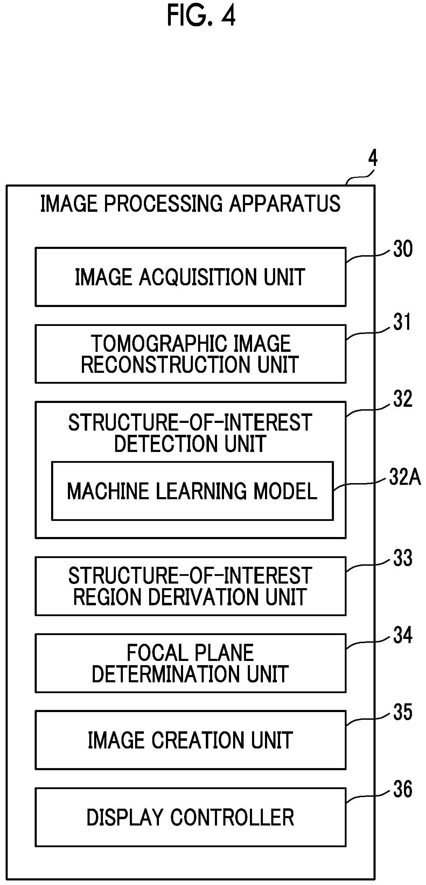

Hereinafter, a functional configuration of the image processing apparatus 4 according to the present embodiment will be described. Fig. 4 is a diagram illustrating a functional configuration of the image processing apparatus 4 according to the present embodiment.

As illustrated in Fig. 4, the image processing apparatus 4 according to the present embodiment comprises an image acquisition unit 30, a tomographic image reconstruction unit 31, a structure-of-interest detection unit 32, a structure-of-interest region derivation unit 33, a focal plane determination unit 34, an image creation unit 35, and a display controller 36. In addition, in a case where the CPU 21 executes the image processing program 22, the image processing apparatus 4 functions as the image acquisition unit 30, the tomographic image reconstruction unit 31, the structure-of-interest detection unit 32, the structure-of-interest region derivation unit 33, the focal plane determination unit 34, the image creation unit 35, and the display controller 36.

The image acquisition unit 30 acquires a plurality of projection images acquired by causing the console 2 to perform tomosynthesis imaging on the breast M by the mammography apparatus 1. The image acquisition unit 30 acquires the projection images from the console 2 or the image storage system 3 via the network I/F 27. Further, the tomographic image reconstruction unit 31 reconstructs a plurality of tomographic images from the projection images acquired by the image acquisition unit 30.

Here, the tomosynthesis imaging and the generation of the tomographic image will be described. In a case of performing the tomosynthesis imaging for generating a tomographic image, the console 2 acquires a plurality of projection images Gi (i = 1 to n, where n is the number of radiation source positions and is, for example, n=15) at a plurality of radiation source positions by moving the radiation source 16 by rotating the arm part 12 around the rotation shaft 11, irradiating the breast M as a subject with radiation at a plurality of radiation source positions obtained by the movement of the radiation source 16 according to predetermined imaging conditions for tomosynthesis imaging, and detecting the radiation passing through the breast M by the radiation detector 15.

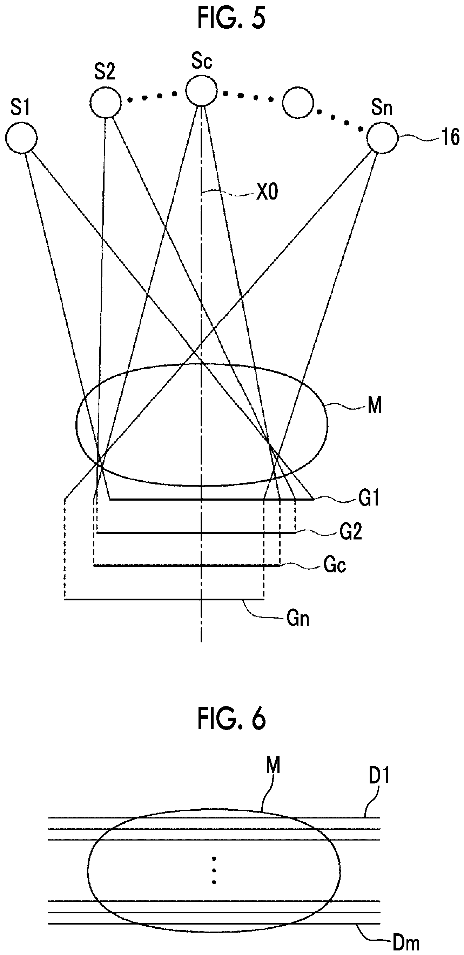

Fig. 5 is a diagram for describing the acquisition of the projection images Gi. As illustrated in Fig. 5, the radiation source 16 is moved to each of radiation source positions S1, S2, ···, and Sn. The radiation source 16 drives and irradiates the breast M with radiation at each of the radiation source positions. The radiation detector 15 detects X-rays passing through the breast M, and thus the projection images G1, G2, ···, and Gn corresponding to the radiation source positions S1 to Sn are acquired. At each of the radiation source positions S1 to Sn, the breast M is irradiated with the same dose of radiation.

In Fig. 5, the radiation source position Sc is a radiation source position at which the optical axis X0 of the radiation emitted from the radiation source 16 is orthogonal to the detection surface 15A of the radiation detector 15. The radiation source position Sc is referred to as a reference radiation source position Sc.

In addition, the tomographic image reconstruction unit 31 generates a tomographic image in which the desired tomographic planes of the breast M are emphasized by reconstructing the plurality of projection images Gi. Specifically, the image processing apparatus 4 generates a plurality of tomographic images Dj (j = 1 to m) on each of the plurality of tomographic planes of the breast M as illustrated in Fig. 6 by reconstructing the plurality of projection images Gi using a known back projection method, such as a simple back projection method or a filtered back projection method. In this case, a three-dimensional coordinate position in a three-dimensional space including the breast M is set, pixel values of the corresponding pixels in the plurality of projection images Gi are reconstructed with respect to the set three-dimensional coordinate position, and pixel values at the coordinate positions are calculated.

The structure-of-interest detection unit 32 detects a structure of interest from the plurality of tomographic images Dj. In the present embodiment, a tumor, a spicula, and a calcification included in the breast M are detected as the structure of interest.

The structure-of-interest detection unit 32 according to the present embodiment detects a structure-of-interest by using a machine learning model 32A that is configured with a neural network obtained by performing machine learning, such as deep learning, using training data so as to extract a structure-of-interest from each of the tomographic images Dj. As the machine learning model 32A, for example, a well-known neural network, such as a convolutional neural network (CNN) or a support vector machine (SVM), can be used.

As described above, in the present embodiment, the structure of interest is detected by the machine learning model 32A. On the other hand, the present disclosure is not limited thereto. For example, an aspect in which the structure of interest is detected from the tomographic image Dj using a known computer aided diagnosis (that is, CAD) algorithm may be used. In the algorithm by the CAD, the probability (likelihood) indicating that the pixel in the tomographic image Dj is the structure of interest is derived, and the pixel in which the probability is equal to or higher than the predetermined threshold value is detected as the structure of interest. An algorithm by CAD is prepared for each type of the structures of interest. In a case where the algorithm by the CAD is applied in the present embodiment, a CAD algorithm for tumor detection, a CAD algorithm for spicula detection, and a CAD algorithm for calcification detection are prepared.

In addition, the detection of the structure of interest is not limited to detection using the machine learning model 32A or detection using the CAD. For example, the structure of interest may be detected from the tomographic image Dj by performing pattern matching using a template image of a structure of interest to be detected or a filtering process using a filter for detecting the structure of interest. Here, in a case where the pattern matching is applied, an image that is already acquired for the type of the structure of interest to be extracted is applied as the template image. In addition, in the present embodiment, the structure of interest is detected using only the machine learning model 32A, but the present disclosure is not limited thereto. For example, a form in which the structure of interest is detected by using both the machine learning model 32A and the pattern matching may be used. Further, in this form, a method of detecting the structure of interest may be switched according to the type of the structure of interest to be detected.

Further, as described above, in the present embodiment, all of the tumor, the spicula, and the calcification are applied as the structures of interest to be detected, but the present disclosure is not limited thereto. For example, one of the tumor, the spicula, and the calcification, or a combination of two of the tumor, the spicula, and the calcification may be applied as the structures of interest to be detected.

The structure-of-interest region derivation unit 33 derives a region of the projection image Gi corresponding to the structure of interest that is detected by the structure-of-interest detection unit 32. The structure-of-interest region derivation unit 33 according to the present embodiment derives a two-dimensional coordinate position of the structure of interest in the corresponding projection image Gi, from the position of the structure of interest in the tomographic image Dj that is detected by the structure-of-interest detection unit 32, the position of the structure of interest in the tomographic image Dj with respect to a stacking direction (a depth direction in which the tomographic planes are arranged), the position of the radiation source 16 when performing imaging of the corresponding projection image Gi, and the position of the detection surface 15A of the radiation detector 15.

That is, the position of the structure of interest in the tomographic image Dj is detected by the structure-of-interest detection unit 32, and the tomographic image Dj and the positions of the radiation source 16 and the detection surface 15A are specified in advance. Therefore, the structure-of-interest region derivation unit 33 according to the present embodiment derives a two-dimensional coordinate position (hereinafter, referred to as a "structure-of-interest position") of the structure of interest in the projection image Gi in a case where the structure of interest is projected onto the detection surface 15A of the radiation detector 15 by the radiation emitted from the radiation source 16, by performing calculation using the positional relationship.

The focal plane determination unit 34 determines whether or not the tomographic image Dj from which the structure of interest is detected by the structure-of-interest detection unit 32 is a tomographic image corresponding to a tomographic plane on which the structure of interest is actually located (hereinafter, referred to as a "focal plane"), by using the region of the projection image Gi derived by the structure-of-interest region derivation unit 33, that is, the structure-of-interest position in each of the projection images Gi.

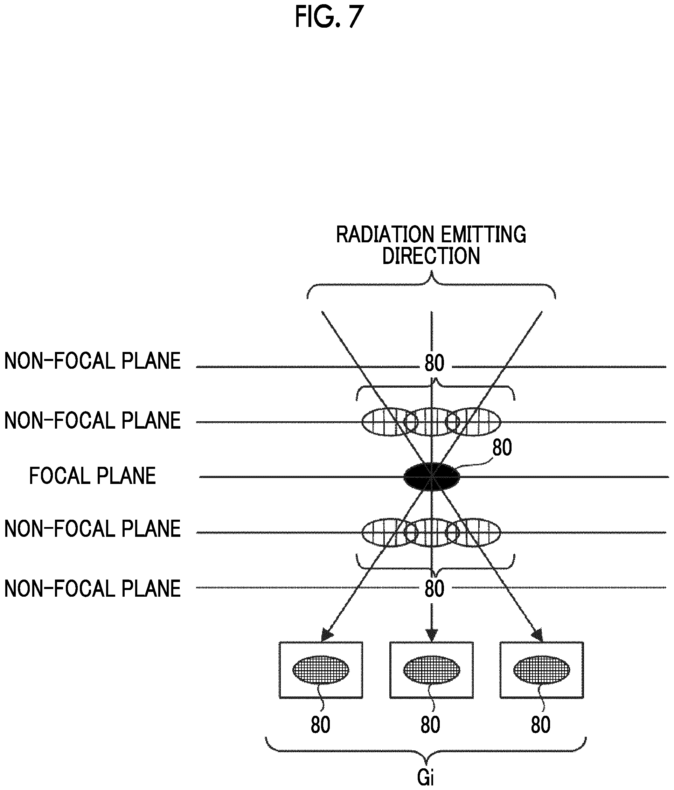

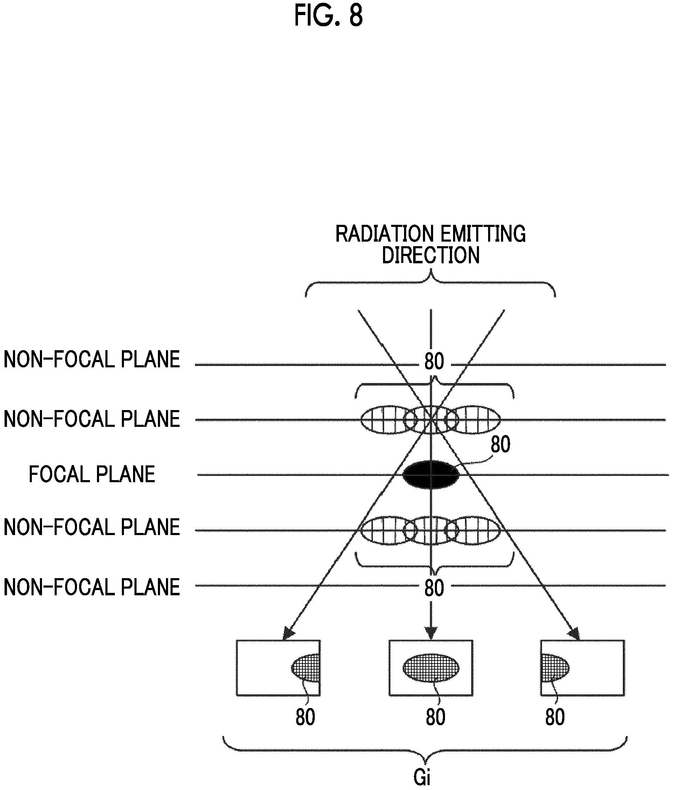

That is, in a case where the tomographic image from which the structure of interest is detected is a tomographic image corresponding to the focal plane, as illustrated in Fig. 7 as an example, the structures of interest 80 appear at substantially the same position in each of the projection images Gi. On the other hand, in a case where the tomographic image from which the structure of interest is detected is a tomographic image corresponding to a plane other than the focal plane (hereinafter, referred to as a "non-focal plane"), the focus is shifted. Therefore, as illustrated in Fig. 8 as an example, the structures of interest 80 appearing in each of the projection images Gi are in a shifted state. Note that Fig. 7 and Fig. 8 are diagrams illustrating a method of determining the tomographic image corresponding to the focal plane according to the present embodiment.

Therefore, the focal plane determination unit 34 according to the present embodiment determines whether or not the tomographic image corresponds to the focal plane by determining whether or not the projection images Gi corresponding to the tomographic image from which the structure of interest is detected are similar to each other.

The focal plane determination unit 34 according to the present embodiment cuts out, from each of the projection images Gi (hereinafter, referred to as "target projection images") corresponding to the tomographic image from which the structure of interest is detected, a rectangular region that includes the structure-of-interest position derived by the structure-of-interest region derivation unit 33 and has the same area (hereinafter, referred to as a "cut-out region"). In addition, the focal plane determination unit 34 according to the present embodiment determines whether or not the corresponding tomographic image corresponds to the focal plane by determining whether or not the cut-out regions in the target projection images are similar to each other.

The focal plane determination unit 34 according to the present embodiment performs determination as to whether or not the cut-out regions in the target projection images are similar to each other, by using an average value of similarities between the cut-out regions. In addition, in the present embodiment, as the similarity, a correlation value between the corresponding cut-out regions is applied.

As a correlation value between images, various values that can be relatively easily calculated, such as a sum of absolute difference (SAD), a sum of squared difference (SSD), a normalized cross-correlation (NCC), and a zero-mean normalized cross-correlation (ZNCC), can be applied. On the other hand, in the present embodiment, SAD is applied among these values. That is, the focal plane determination unit 34 according to the present embodiment calculates, as the correlation value, a sum value of absolute values of differences in brightness values of the corresponding pixels between the cut-out regions for all pixels. Here, the present disclosure is not limited to the form. A form in which another correlation value such as SSD or NCC is applied may be adopted. Of course, as the similarity, a value other than the correlation value that indicates a similarity between the cut-out regions may be applied.

In addition, the focal plane determination unit 34 according to the present embodiment determines, for each type of the structures of interest, that the tomographic image at a height at which the corresponding similarity (here, the correlation value) has a maximum value, among the tomographic images from which the structure of interest is detected and which are at heights, is the tomographic image corresponding to the focal plane. Thereby, it is possible to determine the tomographic image with a high quality for each type of the structures of interest.

The image creation unit 35 creates a synthesized two-dimensional image from the plurality of tomographic images Dj by using a result of the determination by the focal plane determination unit 34.

The image creation unit 35 according to the present embodiment creates a synthesized two-dimensional image by setting a weight value (for example, 0.9) of a tomographic image, which is determined as corresponding to the focal plane by the focal plane determination unit 34, to a value larger than a weight value (for example, 0.1) of a tomographic image other than the tomographic image corresponding to the focal plane, that is, a tomographic image corresponding to the non-focal plane and taking a weighted average for each pixel. Here, the method of creating the synthesized two-dimensional image is not limited to such an averaging method, and an addition method, a maximum intensity projection method, a minimum intensity projection method, or the like may be applied as a method of creating a synthesized two-dimensional image.

For example, in a case where the minimum intensity projection method is applied as a method of creating a synthesized two-dimensional image, a form in which only tomographic images obtained by excluding the tomographic image corresponding to the non-focal plane from the tomographic images to be projected are used as targets may be adopted.

As described above, in the present embodiment, a synthesized two-dimensional image is applied as an image created by the image creation unit 34. On the other hand, the present disclosure is not limited thereto. For example, a form in which a slab image that is an image including thickness information is applied as an image created by the image creation unit 34 may be adopted. As a form example in this case, a form in which a slab image is created using only the tomographic image corresponding to the focal plane may be adopted.

The display controller 36 displays the synthesized two-dimensional image generated by the image creation unit 35 on the display 24. The synthesized two-dimensional image is created by setting a weight value for the tomographic image corresponding to the focal plane of the structure of interest to be larger than weight values for the other tomographic images. Therefore, the synthesized two-dimensional image displayed here is an image in which artifacts are suppressed and the structure of interest is emphasized and which has an extremely high quality.

The image created by the image creation unit 35 is not limited to the synthesized two-dimensional image and the slab image described above, and may be an image for performing display of linking the synthesized two-dimensional image created from the plurality of tomographic images Dj with the corresponding tomographic images Dj.

That is, in general, the number of the tomographic images may be equal to or larger than 100 depending on the application. Therefore, in a case of reading the tomographic images, there is a problem that a relatively long reading time is required.

Therefore, in this form, the CPU 21 first creates a synthesized two-dimensional image as described above, and displays the synthesized two-dimensional image. Accordingly, an operator refers to the displayed synthesized two-dimensional image, and in a case where a lesion is suspected in the structure of interest displayed on the synthesized two-dimensional image, designates the structure of interest. In response to the designation, the CPU 21 displays the tomographic image including the designated structure of interest. Thereby, a reading time of the tomographic image can be significantly shortened.

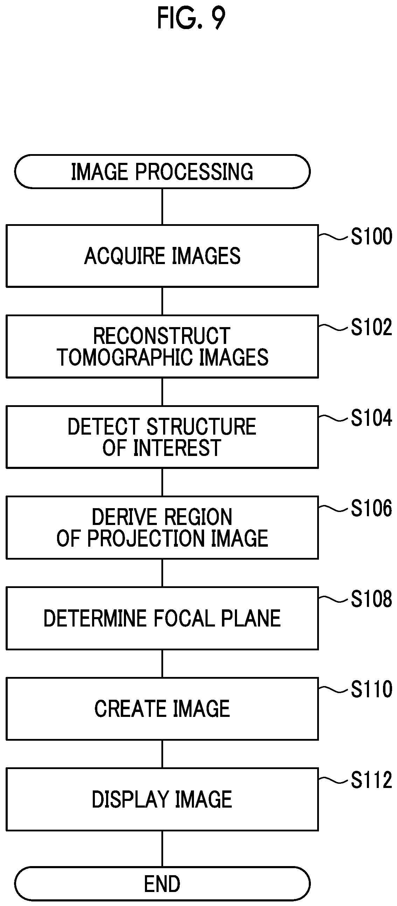

Next, processing performed in the present embodiment will be described. Fig. 9 is a flowchart illustrating image processing performed in the present embodiment. It is assumed that a plurality of projection images Gi are acquired in advance and stored in the storage 23.

In a case where the input device 25 receives an instruction to start the processing from the operator, execution of the image processing program 22 is started, and thus, the image processing illustrated in Fig. 9 is started.

First, the image acquisition unit 30 acquires the projection images Gi by reading the projection images Gi from the storage 23, the projection images Gi being obtained by imaging the subject (in the present embodiment, the breast M) with the radiation emitted from the radiation source 16 at a plurality of angles (step S100). Next, the tomographic image reconstruction unit 31 reconstructs the plurality of tomographic images Dj from the acquired projection images Gi (step S102).

Next, the structure-of-interest detection unit 32 detects a structure of interest from the plurality of reconstructed tomographic images Dj (step S104). Next, the structure-of-interest region derivation unit 33 derives a region of the projection image corresponding to the detected structure of interest (step S 106). Next, the focal plane determination unit 34 determines whether or not the tomographic image from which the structure of interest is detected is a tomographic image corresponding to the focal plane by using the derived region of the projection image (step S108).

In addition, the image creation unit 35 creates a synthesized two-dimensional image from the plurality of tomographic images Dj by using a result of the determination (step S110). Subsequently, the display controller 36 displays the synthesized two-dimensional image on the display 24 (step S 112), and the processing is ended.

As described above, the image processing apparatus according to the present embodiment acquires the projection images obtained by imaging the subject with the radiation at a plurality of angles, reconstructs the plurality of tomographic images from the acquired projection images, detects the structure of interest from the plurality of reconstructed tomographic images, derives the region of the projection image corresponding to the detected structure of interest, and determines whether or not the tomographic image from which the structure of interest is detected is a tomographic image corresponding to the focal plane by using the derived region of the projection image. Therefore, it is possible to perform the determination with higher accuracy as compared with a case of determining a tomographic image including the structure of interest and corresponding to the focal plane from the tomographic images.

Further, with the image processing apparatus according to the present embodiment, a synthesized two-dimensional image is created from the plurality of tomographic images by using the result of the determination. Therefore, it is possible to create a synthesized two-dimensional image in which the structure of interest located on the focal plane is emphasized, and as a result, it is possible to create a synthesized two-dimensional image with a higher quality.

Further, in the image processing apparatus according to the present embodiment, an aspect in which a slab image is created from the plurality of tomographic images by using a result of the determination may be also adopted. Therefore, according to the aspect, it is possible to create a slab image in which the structure of interest located on the focal plane is emphasized, and as a result, it is possible to create a slab image with a higher quality.

Further, in the image processing apparatus according to the present embodiment, an aspect in which display of linking the synthesized two-dimensional image created from the plurality of tomographic images and the corresponding tomographic image is performed by using the result of the determination may be also adopted. Therefore, according to the aspect, the display of linking the images can be more effectively performed.

In addition, in the image processing apparatus according to the present embodiment, the structure of interest is detected by the machine learning model. Therefore, it is possible to detect the structure of interest with higher accuracy, as compared with a case of detecting the structure of interest without using the machine learning model.

Further, in the image processing apparatus according to the present embodiment, the determination is performed using the similarity between the regions of the corresponding projection images, and the correlation value that can be relatively easily calculated is applied as the similarity. Therefore, the determination can be more simply performed, as compared with a case where the determination is performed without using the correlation value.

Further, in the image processing apparatus according to the present embodiment, for each type of the structures of interest, it is determined that the tomographic image at a height at which the corresponding similarity has a maximum value, among the tomographic images from which the structure of interest is detected and which are at heights, is the tomographic image corresponding to the focal plane. Therefore, it is possible to determine the tomographic image with a high quality for each type of the structures of interest.

Further, in the image processing apparatus according to the present embodiment, at least one of a tumor, a spicula, or a calcification is applied as the structure of interest. Therefore, it is possible to display the synthesized image in which the applied structure of interest is emphasized.

In the embodiment, a case where the CPU 21 provided in the image processing apparatus 4 is applied as the processor according to the technology of the present disclosure has been described. On the other hand, the present disclosure is not limited thereto. For example, the CPU provided in any of the mammography apparatus 1, the console 2, and the image storage system 3 may be applied as a processor according to the technology of the present disclosure.

In addition, in the embodiment, for example, as hardware structures of processing units that execute various kinds of processing, such as the image acquisition unit 30, the tomographic image reconstruction unit 31, the structure-of-interest detection unit 32, the structure-of-interest region derivation unit 33, the focal plane determination unit 34, the image creation unit 35, and the display controller 36, the following various processors can be used. As described above, the various processors include, in addition to the CPU that is a general-purpose processor that executes software (program) to function as various processing units, a programmable logic device (PLD) that is a processor of which a circuit configuration can be changed after manufacture, such as a field programmable gate array (FPGA), and a dedicated electric circuit that is a processor having a circuit configuration that is designed for exclusive use in order to execute a specific process, such as an application specific integrated circuit (ASIC).

One processing unit may be configured of one of the various processors, or configured of a combination of the same or different kinds of two or more processors (for example, a combination of a plurality of FPGAs or a combination of the CPU and the FPGA). In addition, a plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that implements the function of the entire system including the plurality of processing units by one integrated circuit (IC) chip is used may be adopted. In this way, various processing units are formed using one or more of the above-mentioned various processors as hardware structures.

Furthermore, as the hardware structure of the various processors, more specifically, an electrical circuit (circuitry) in which circuit elements such as semiconductor elements are combined can be used.

In addition, in the embodiment described above, the aspect in which the image processing program 22 is stored (installed) in advance in the storage 23 of the image processing apparatus 4 has been described, but the present disclosure is not limited thereto. The image processing program 22 may be provided in a form recorded in a recording medium such as a compact disc read-only memory (CD-ROM), a digital versatile disc read-only memory (DVD-ROM), and a universal serial bus (USB) memory. In addition, the image processing program 22 may be configured to be downloaded from an external apparatus via a network.

From the above description, the invention described in following Appendices can be understood.

### [Appendix 1]

An image processing apparatus comprising:
at least one processor,
in which the processor is configured to:
   acquire projection images obtained by imaging a subject with radiation at a plurality of angles;
   reconstruct a plurality of tomographic images from the acquired projection images;
   detect a structure of interest from the plurality of reconstructed tomographic images;
   derive a region of the projection image corresponding to the detected structure of interest; and
   determine, by using the derived region of the projection image, whether or not the tomographic image from which the structure of interest is detected is a tomographic image corresponding to a focal plane of the structure of interest.

### [Appendix 2]

The image processing apparatus according to Appendix 1,
in which the processor is configured to: create a synthesized two-dimensional image from the plurality of tomographic images by using a result of the determination.

### [Appendix 3]

The image processing apparatus according to Appendix 1 or Appendix 2,
in which the processor is configured to: create a slab image from the plurality of tomographic images by using a result of the determination.

### [Appendix 4]

The image processing apparatus according to any one of Appendixes 1 to 3,
in which the processor is configured to: perform display of linking a synthesized two-dimensional image created from the plurality of tomographic images and the corresponding tomographic image by using a result of the determination.

### [Appendix 5]

The image processing apparatus according to any one of Appendixes 1 to 4,
in which the processor is configured to: detect the structure of interest by using at least one of a machine learning model or pattern matching using a template image of the target structure of interest.

### [Appendix 6]

The image processing apparatus according to any one of Appendixes 1 to 5,
in which the processor is configured to: perform the determination by using a similarity between the regions of the corresponding projection images.

### [Appendix 7]

The image processing apparatus according to Appendix 6,
in which the similarity is a correlation value between the regions of the corresponding projection images.

### [Appendix 8]

The image processing apparatus according to Appendix 6 or Appendix 7,
in which the processor is configured to: determine, for each type of the structure of interest, that the tomographic image at a height at which the corresponding similarity has a maximum value, among the tomographic images from which the structure of interest is detected and which are at heights, is the tomographic image corresponding to the focal plane.

### [Appendix 9]

The image processing apparatus according to any one of Appendixes 1 to 8,
in which the structure of interest is at least one of a tumor, a spicula, or a calcification.

### [Appendix 10]

A radiography system comprising:
the image processing apparatus according to any one of Appendixes 1 to 9; and
a mammography apparatus that acquires projection images to be used by the image processing apparatus.

### [Appendix 11]

A program causing a computer to execute a process comprising:
acquiring projection images obtained by imaging a subject with radiation at a plurality of angles;
reconstructing a plurality of tomographic images from the acquired projection images;
detecting a structure of interest from the plurality of reconstructed tomographic images;
deriving a region of the projection image corresponding to the detected structure of interest; and
determining, by using the derived region of the projection image, whether or not the tomographic image from which the structure of interest is detected is a tomographic image corresponding to a focal plane of the structure of interest.

## Claims

1. An image processing apparatus comprising:
at least one processor that is configured to:
acquire projection images obtained by imaging a subject with radiation at a plurality of angles;
reconstruct a plurality of tomographic images from the acquired projection images;
detect a structure of interest from the plurality of reconstructed tomographic images;
derive a region of the projection image corresponding to the detected structure of interest; and
determine, by using the derived region of the projection image, whether or not the tomographic image from which the structure of interest is detected is a tomographic image corresponding to a focal plane of the structure of interest.

2. The image processing apparatus according to claim 1,
wherein the at least one processor is configured to: generate a synthesized two-dimensional image from the plurality of tomographic images by using a result of determination.

3. The image processing apparatus according to claim 1 or 2,
wherein the at least one processor is configured to: generate a slab image from the plurality of tomographic images by using a result of determination.

4. The image processing apparatus according to any one of claims 1 to 3,
wherein the at least one processor is configured to: perform display of linking a synthesized two-dimensional image generated from the plurality of tomographic images and the corresponding tomographic image by using a result of determination.

5. The image processing apparatus according to any one of claims 1 to 4,
wherein the at least one processor is configured to: detect the structure of interest by using at least one of a machine learning model or pattern matching using a template image of the target structure of interest.

6. The image processing apparatus according to any one of claims 1 to 5,
wherein the at least one processor is configured to: perform the determination by using a similarity between the regions of the corresponding projection images.

7. The image processing apparatus according to claim 6,
wherein the similarity is a correlation value between the regions of the corresponding projection images.

8. The image processing apparatus according to claim 6 or 7,
wherein the at least one processor is configured to: determine, for each type of the structure of interest, that the tomographic image at a height at which the corresponding similarity has a maximum value, among the tomographic images from which the structure of interest is detected and which are at heights, is the tomographic image corresponding to the focal plane.

9. The image processing apparatus according to any one of claims 1 to 8,
wherein the structure of interest is at least one of a tumor, a spicula, or a calcification.

10. A radiography system comprising:
the image processing apparatus according to any one of claims 1 to 9; and
a mammography apparatus that acquires projection images to be used by the image processing apparatus.

11. A non-transitory storage medium storing a program that causes a computer to execute an image processing, the image processing comprising:
acquiring projection images obtained by imaging a subject with radiation at a plurality of angles;
reconstructing a plurality of tomographic images from the acquired projection images;
detecting a structure of interest from the plurality of reconstructed tomographic images;
deriving a region of the projection image corresponding to the detected structure of interest; and
determining, by using the derived region of the projection image, whether or not the tomographic image from which the structure of interest is detected is a tomographic image corresponding to a focal plane of the structure of interest.

12. An image processing method executed by a computer, the method comprising:
acquiring projection images obtained by imaging a subject with radiation at a plurality of angles;
reconstructing a plurality of tomographic images from the acquired projection images;
detecting a structure of interest from the plurality of reconstructed tomographic images;
deriving a region of the projection image corresponding to the detected structure of interest; and
determining, by using the derived region of the projection image, whether or not the tomographic image from which the structure of interest is detected is a tomographic image corresponding to a focal plane of the structure of interest.
